# EUROPEAN PATENT SPECIFICATION

(11) **EP 1 994 051 B1**
(45) Date of publication and mention of the grant of the patent: **26.10.2011**
(21) Application number: 07708193.3
(22) Date of filing: 01.02.2007
(51) Int. Cl.: C07K 14/435, C12Q 1/42

(54) **GENE ENCODING TREHALOSE-6-PHOSPHATE PHOSPHATASE AND USE THEREOF**
FÜR TREHALOSE-6-PHOSPHATPHOSPHATASE CODIERENDES GEN UND VERWENDUNG DAVON
GÈNE CODANT POUR LA TRÉHALOSE-6-PHOSPHATE PHOSPHATASE ET APPLICATIONS

(30) Priority: 28.02.2006 JP 2006054191
(43) Date of publication of application: 26.11.2008
(73) Proprietor: Suntory Holdings Limited, Kita-ku, Osaka-shi, Osaka 530-8203 (JP)
(72) Inventor: NAKAO, Yoshihiro c/o SUNTORY LIMITED,, Shimamoto-cho, Mishima-gun, Osaka;6188503 (JP); KODAMA, Yukiko c/o SUNTORY LIMITED,, Shimamoto-cho, Mishima-gun, Osaka;6188503 (JP); SHIMONAGA, Tomoko c/o Suntory Limited, Mishima-gun, Osaka;6188503 (JP)
(74) Representative: Vossius & Partner
(86) International application number: PCT/JP2007/052167
(87) International publication number: WO 2007/099749

(56) References cited:
- WO-A-01/16357
- DATABASE EMBL [Online] 15 March 1993 (1993-03-15), "S.cerevisiae TPS2 gene for trehalose-6-phosphate synthase/phosphatase 100 kDa subunit" XP002430279 retrieved from EBI accession no. EMBL:X70694 Database accession no. X70694 -& VIRGILIO DE C ET AL: "DISRUPTION OF TPS2, THE GENE ENCODING THE 100-KDA SUBUNIT OF THE TREHALOSE-6-PHOSPHATE SYNTHASE/PHOSPHATASE COMPLEX IN SACCHAROMYCESCEREVISIAE, CAUSES ACCUMULATION OF TREHALOSE-6-PHOSPHATE AND LOSS OF TREHALOSE-6-PHOSPHATE PHOSPHATASE ACTIVITY" EUROPEAN JOURNAL OF BIOCHEMISTRY, BERLIN, DE, vol. 212, no. 2, March 1993 (1993-03), pages 315-323, XP002041188 ISSN: 0014-2956 cited in the application
- HABIBUR RAHMAN PRAMANIK M ET AL: "Functional Identification of a Trehalose 6-phosphate Phosphatase Gene that is Involved in Transient Induction of Trehalose Biosynthesis during Chilling Stress in Rice" PLANT MOLECULAR BIOLOGY, KLUWER ACADEMIC PUBLISHERS, DO, vol. 58, no. 6, 1 August 2005 (2005-08-01), pages 751-762, XP019262735 ISSN: 1573-5028
- KANDROR OLGA ET AL: "Yeast adapt to near-freezing temperatures by STRE/Msn2,4-dependent induction of trehalose synthesis and certain molecular chaperones" MOLECULAR CELL, vol. 13, no. 6, 26 March 2004 (2004-03-26), pages 771-781, XP002430276 ISSN: 1097-2765
- KWON HAWK-BIN ET AL: "Cloning and characterization of genes encoding trehalose-6-phosphate synthase (TPS1) and trehalose-6-phosphate phosphatase (TPS2) from Zygosaccharomyces rouxii." FEMS YEAST RESEARCH, vol. 3, no. 4, June 2003 (2003-06), pages 433-440, XP002430277 ISSN: 1567-1356

## Description

### TECHNICAL FIELD

The present invention relates to a gene encoding trehalose-6-phosphate phosphatase and use thereof, in particular, a yeast for practical use with superior resistance property to dryness, alcoholic beverages produced with said yeast, and a method for producing said beverages. More particularly, the present invention relates to a yeast, whose resistance property to dryness is enhanced by amplifying expression level of TPS2 gene encoding a protein Tps2p having a trehalose-6-phosphate phosphatase activity in brewer's yeast, especially non-ScTPS2 gene specific to a lager brewing yeast and to a method for producing alcoholic beverages with said yeast, etc. Further, the yeast of the present invention is useful as a baker's yeast or an industrial yeast as well

### BACKGROUND ART

Beer brewing is characterized by a process recovering yeasts after fermentation and using the recovered yeasts at the subsequent fermentation, which is called "Renjo". The yeasts are stored in the presence of ethanol in a tank whose temperature is kept at approximately 0 to 3°C. When the yeasts die during the storage, not only the next fermentation process is interfered, but also constituents of the yeast cells released by cell lysis may impart unfavorable taste to products. Therefore, it is very important for allowing some variance to design production process and for stable production of quality products to use yeasts with superior resistant property to low-temperature storage.

"Renjo" may be terminated at a certain times of fermentation is carried out The number of times of "Renjo" may vary according to fermentation conditions or properties of yeasts used in the process. A process to develop yeasts for fermentation freshly is called propagation. Yeasts are subcultured several times enlarging scales of culture successively during the propagation process. Because propagation process requires from several days to several weeks, it brings great advantages in production efficiency if term of the process is shortened or yeast cells which are large-scale pre-cultured are able to be stored stably for extended period of time at low temperature or under dry condition.

Concerning a method for producing dry yeast maintaining high viable cell ratio, improvement of drying equipment, or improvement of manufacturing conditions such as temperature or addition of emulsifiers, etc., have been made. For example, L-drying method is not practical to be used at industrial production scale because, though it can maintain extremely high viable cell ratio, but at the same time it takes a lot of time and cost

Regarding low-temperature resistance of yeast, some experiments designed to improve refrigeration-resistant property mainly of baker's yeast were reported. This is because Saccharomyces cerevisiae, which is a baker's yeast, has poor low-temperature storage property in comparison with brewer's yeast for beer or sake, which can ferment at low temperature. For example, baker's yeasts having refrigeration-resistant property and drying-resistant property were found out mainly by screening methods in Japanese Patent Application Laid-open No. H-11-155559 and Japanese Patent Application Laid-open No. 2003-304864. Further, regarding examples utilizing genetic engineering techniques, trehalose highly accumulating strains by disruption of NTH1, which is a trehalase gene, is reported in Japanese Patent Application Laid-open No. H10-117771 and a strain highly accumulating specific amino acids such as arginine by disruption of CAR1, which is an arginase gene, is reported in Japanese Patent Application Laid-open No. 2001-238665. The TPS2 from different fungi have been disclosed in EM_FUN:X70694, WO 01/16357, Kandror et al. (Mol. Cell., 2004, 13:771-81) and Kwon et al. (FEMS Yeast Research, 2003, 3:433-440). A putative trehalose-6-phosphate phosphatase was isolated from rice in Pramanik et al. (Plant Mol. Biol., 2005, 58:751-62).

### DISCLOSURE OF INVENTION

Under the above situations, there has been a need to make high-efficiency production of alcoholic beverages or useful materials possible by using a gene encoding a protein responsible for drying bf brewery yeast and said protein.

The present inventors made extensive studies to solve the above problems and as a result, succeeded in identifying and isolating a gene encoding trehalose-6-phosphate phosphatase from beer yeast. Moreover, the present inventors produced transformed yeast in which the obtained gene was expressed to verify that drying-resistant property can be actually improved, thereby completing the present invention.

Thus, the present invention relates to a gene encoding a trehalose-6-phosphate phosphatase of brewery yeast, to a protein encoded by said gene, to a transformed yeast in which the expression of said gene is controlled, to a method for enhancing drying-resistant property of yeast using a yeast in which the expression of said gene is controlled, or the like. More specifically, the present invention provides the following polynucleotides, a vector comprising said polynucleotide, a transformed yeast introduced with said vector, a method for producing alcoholic beverages by using said transformed yeast, and the like.
(1) A polynucleotide selected from the group consisting of:
   (a) a polynucleotide comprising a polynucleotide consisting of the nucleotide sequence of SEQ ID NO: 1;
   (b) a polynucleotide comprising a polynucleotide encoding a protein consisting of the amino acid sequence of SEQ ID NO: 2;
   (c) a polynucleotide comprising a polynucleotide encoding a protein consisting of the amino acid sequence of SEQ ID NO: 2 in which one to 26 amino acids thereof are deleted, substituted, inserted and/or added, and having a trehalose-6-phosphate dephosphorylation activity, and
   (d) a polynucleotide comprising a polynucleotide encoding a protein having an amino acid sequence having 97% or higher identity with the amino acid sequence of SEQ ID NO: 2, and said protein having a trehalose-6-phosphate dephosphorylation activity.
(2) The polynucleotide according to (1) above selected from the group consisting of
   (g) a polynucleotide comprising a polynucleotide encoding a protein consisting of the amino acid sequence of SEQ ID NO: 2, or encoding the amino acid sequence of SEQ ID NO: 2 in which 1 to 8 amino acids thereof are deleted, substituted, inserted, and/or added, and wherein said protein has a trehalose-6-phosphate dephosphorylation activity, and
   (h) a polynucleotide comprising a polynucleotide encoding a protein having 99% or higher identity with the amino acid sequence of SEQ ID NO: 2, and having a trehalose-6-phosphate dephosphorylation activity.
(3) The polynucleotide according to (1) above comprising a polynucleotide consisting of the nucleotide sequence of SEQ ID NO: 1.
(4) The polynucleotide according to (1) above comprising a polynucleotide encoding a protein consisting of the amino acid sequence of SEQ ID NO: 2.
(5) The polynucleotide according to any one of (1) to (4) above, wherein the polynucleotide is DNA
(6) A protein encoded by the polynucleotides according to any one of (1) to (5) above.
(7) A vector containing the polynucleotide according to any one of (1) to (5) above.
(8) A yeast into which the vector according to (7) above has been introduced.
(9) The yeast (yeast for practical use) according to (8) above, wherein drying-resistant property is increased. The "yeast for practical use" means that a yeast which possesses practical value such as brewer's (brewery) yeast, baker's yeast or industrial yeast, etc.
(10) A method for producing an alcoholic beverage by using the yeast according to any one of (8) to (9) above.
(11) A method for assessing a test yeast for its drying-resistant property, comprising using a primer or probe designed based on the nucleotide sequence of a gene having the nucleotide sequence of SEQ ID NO: 1 and encoding a trehalose-6-phosphate phosphatase.
(12) A method for assessing a test yeast for its drying-resistant property, comprising: culturing the test yeast; and measuring the expression level of the gene having the nucleotide sequence of SEQ ID NO: 1 and encoding a trehalose-6-phosphate phosphatase.
(13) A method for selecting a yeast, comprising: culturing test yeasts; quantifying the protein of (6) above or measuring the expression level of the gene having the nucleotide sequence of SEQ ID NO: 1 and encoding a trehalose-6-phosphate phosphatase; and selecting a test yeast having an amount of the protein or the gene expression level according to favorable drying-resistant property.
(22) A method for producing an alcoholic beverage comprising: conducting fermentation using the yeast according to (8) or (9) above or a yeast selected by the methods according to (13) above.

The transformed yeast of the present invention is able to keep high viable cell ratio during dry storage. Therefore, when it is used for brewing and so on, painfulness of conserving yeast can be eliminated. Further, it is expected to contribute to quality stabilization. Moreover, dry yeast is suitable for long-storage, and it is very advantageous to distribution or transportation due to its reduced weight. It is also useful as microorganisms for industrial application such as industrial alcohol production or production of useful proteins. The yeast of the present invention also useful as an industrial yeast as well.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScTPS2 gene in yeasts upon beer fermentation test. The horizontal axis represents fermentation time while the vertical axis represents the intensity of detected signal.
Figure 4 shows the result of drying-resistant property test of parent strain and non-ScTPS2 highly expressed strain.

### BEST MODES FOR CARRYING OUT THE INVENTION

The present inventors isolated and identified non-ScTPS2 gene encoding a trehalose-6-phosphate phosphatase of brewery yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide of the nucleotide sequence of SEQ ID NO: 1; and. (b) a polynucleotide comprising a polynucleotide encoding a protein of the amino acid sequence of SEQ ID NO: 2. The polynucleotide can be DNA or RNA.

The target polynucleotide of the present invention is not limited to the polynucleotide encoding a protein having a trehalose-6-phosphate dephosphorylation activity described above and may include other polynucleotides encoding proteins having equivalent functions to said protein.

Proteins with equivalent functions include, for example, (c) a protein consisting of an amino acid sequence of SEQ ID NO:2 with, for example, 1 to 26, 1 to 25, 1 to 24,1 to 23,1 to 22,1 to 21,1, 1 to 20,1 to 19,1 to 18,1 1 to 17,1 to 16,1 to 15,1 to 14,1 to 13,1 to 12, 1 to 11,1 to 10,1 to 9,1 to 8,1 to 7,1 to 6 (1 to several amino acids), 1 to 5, to 4, 1 to 3, 1 to 2, or 1 amino acid residues thereof being deleted, substituted, inserted and/or added and having a trehalose-6-phosphate dephosphorylation activity. In general, the number of deletions, substitutions, insertions, and/or additions is preferably smaller. In addition, such proteins include (d) a protein having an amino acid sequence with about 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having a trehalose-6-phosphate dephosphorylation activity. In general, the percentage identity is preferably higher.

Trehalose-6-phosphate phosphatase activity may be measured, for example, by a method described in Eur J Biochem. 1993 Mar 1:212(2): 315-23.

Furthermore, disclosed is (e) a polynucleotide comprising a polynucleotide which hybridizes to a polynucleotide consisting of a nucleotide sequence complementary to the nucleotide sequence of SEQ ID NO: 1 under stringent conditions and which encodes a protein having a trehalose-6-phosphate dephosphorylation activity; and (f) a polynucleotide comprising a polynucleotide which hybridizes to a polynucleotide complementary to a nucleotide sequence of encoding a protein of SEQ ID NO: 2 under stringent conditions, and which encodes a protein having a trehalose-6-phosphate dephosphorylation activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or polynucleotide encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "*High stringency conditions*" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for examples, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad Sci USA, 87: 2264-2268, 1990; Proc. Natl Acad Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score =100 and word length =12. When an amino acid sequence is sequenced using BLASTS the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a) to (d), (g) and (h) above. A preferred protein of the present invention comprises an amino acid sequence of SEQ ID NO: 2 with one to 26 amino acids thereof being deleted, substituted, inserted and/or added, and having a trehalose-6-phosphate dephosphorylation activity.

Such protein includes those having an amino acid sequence of SEQ ID NO: 2 with amino acid residues thereof of the number mentioned above being deleted, substituted, inserted, and/or added and having a trehalose-6-phosphate dephosphorylation activity. In addition, such protein includes those having homology as described above with the amino acid sequence of SEQ ID NO: 2 and having a trehalose-6-phosphate dephosphorylation activity.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl Acad Sci. USA 79: 6409 (1982), Gene 34: 315 (1985), Nuc. Acids. Res., 13:4431 (1985), Proc. Natl. Acad. Sci. USA 82: 488 (1985).

Deletion, substitution, insertion and/or addition of one to 26 amino acid residues in an amino acid sequence of the protein of the invention means that one to 26 amino acid residues are deleted, substituted, inserted and/or added at any one to 26 positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and veast transformed with the vector

The present invention then provides a vector comprising the polynucleotide described above. The vector of the present invention is directed to a vector including any of the polynucleotides described in (a) to (d), (g) and (h) above.

Generally, the vector of the present invention may comprise an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule. Further, in order to highly express the protein of the invention, these polynucleotides are preferably introduced in the sense direction to the promoter to promote expression of the polynucleotide (DNA) described in any of (a) to (d), (g) and (h) above.

A vector introduced in the yeast may be any of a multicopy type (YEp type), a single copy type (YCp type), or a chromosome integration type (YIp type). For example, YEp24 (J. R Broach et aL, EXPERIMENTAL MANIPULATION OF GENIE EXPRESSION, Academic Press, New York, 83, 1983) is known as a YEp type vector, YCp50 (M D. Rose et al., Gene 60: 237, 1987) is known as a YCp type vector, and YIp5 (K. Struhl et al., Proc. Natl. Acad Sci. USA, 76: 1035, 1979) is known as a YIp type vector, all of which are readily available.

Promoters/terminators for adjusting gene expression in yeast may be in any combination as long as they function in the yeast for practical use and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a yeast for practical use, for example, a geneticin-resistant gene (G418r), a copper-resistant gene (CUP1) (Marin et al., Proc. Natl Acad Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast (yeast for practical use) that can be used for brewing, for example, brewery yeasts for beer, wine and sake, baker's yeast, yeast for producing industrial alcohol or yeast for producing useful proteins and so on. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae NBRC1951,* NBRC1952, NBRC1953 or NBRC1954, etc., may be used In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan, baker's yeast such as NBRC0555, NBRC1346 or NBRC2043, etc., may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth Enzym., 194: 182 (1990)), a spheroplast method (Proc. Natl. Acad. Sci. USA, 75: 1929(1978)), a lithium acetate method (J. Bacteriology, 153: 163 (1983)), and methods described in Proc. Natl. Acad Sci. USA, 75: 1929 (19.78), METHODS IN YEAST GENETICS, 2000 Edition: A Cold Spring Harbor Laboratory Course ManuaL

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

A yeast having a superior drying-resistant property can be obtained by introducing the vector of the present invention described above to a yeast. Further, a yeast having a superior drying-resistant property can be obtained by selecting a yeast by the yeast assessment method of the present invention described below. The target use of yeasts obtained in the present invention include, for example, but not limited to, brewing alcoholic beverages such as beer, wine, whisky, sake and the like, baking bread, manufacturing useful materials such as industrial alcohol production and production of useful proteins.

In order to produce these products, a known technique can be used except that a yeast for practical use obtained according to the present invention is used in the place of a parent strain. Since starting materials, manufacturing equipment, manufacturing control and the like may be the same as the conventional ones; it can be performed without increasing cost.

### 5. Yeast assessment method of the invention

The present invention relates to a method for assessing a test yeast for its drying-resistant property by using a primer or a probe designed based on a nucleotide sequence of a gene having the nucleotide sequence of SEQ ID NO: 1 and encoding a trehalose-6-phosphate phosphatase. General technique for such assessment method is known and is described in, for example, WO01/040514, Japanese Laid-Open Patent Application No. H8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a trehalose-6-phosphate phosphatase, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the drying-resistant property of yeast as determined by whether the molecular weight of the amplified product is a size that contains the DNA molecule of the specific part In addition, by analyzing the nucleotide sequence of the amplified product, the property may be predicted and/or assessed more precisely.

Moreover, in the present invention, a test yeast is cultured to measure an expression level of the gene encoding a trehalose-6-phosphate phosphatase having the nucleotide sequence of SEQ ID NO: 1 to assess the test yeast for its drying-resistant property.

Measurement of expression level of the gene encoding a trehalose-6-phosphatase can be performed by culturing test yeast and then quantifying mRNA or a protein resulting from the gene. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a trehalose-6-phosphate phosphatase having the nucleotide sequence of SEQ ID NO: 1 are measured to select a test yeast with the gene expression level according to the target trehalose-producing ability, thereby a yeast favorable for brewing desired alcoholic beverages can be selected. In addition, a reference yeast and a test yeast may be cultured so as to measure and compare the expression level of the gene in each of the yeasts, thereby a favorable test yeast can be selected. More specifically, for example, a reference yeast and one or more test yeasts are cultured and an expression level of the gene encoding a trehalose-6-phosphate phosphatase having the nucleotide sequence of SEQ ID NO: 1 is measured in each yeast. By selecting a test yeast with the gene expressed higher than that in the reference yeast, a yeast suitable for brewing desired alcoholic beverages or production of useful materials can be selected.

Alternatively, test yeasts are cultured and a yeast with a high trehalose-producing ability is selected, thereby a yeast suitable for brewing desired alcoholic beverages or production of useful materials can be selected.

In these cases, the test yeasts or the reference yeast may be, for example, a yeast introduced with the vector of the invention, an artificially mutated yeast or a naturally mutated yeast. The trehalose-6-phosphate phosphatase activity can be measured by, for example, a method described in Eur J Biochem. 1993 Mar 1: 212(2): 315-23. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g*., Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeasts (yeasts for practical use), for example, brewery yeasts for beer, wine, sake and the like or baker's yeast, yeast for producing industrial alcohol or yeast for producing useful proteins, etc. More specifically, yeasts such as genus *Saccharomyces* may be used (*e.g., S pastorianus,* S. *cerevisiae,* and *S. carlsbergensis*)*.* According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan, baker's yeast such as NBRC0555, NBRC1346 and NBRC2043, etc., may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples.

### Example 1: Cloning of Gene Encoding Trehalose-6-phosphate phosphatase (non-ScTPS2)

A gene encoding a trehalose-6-phosphate phosphatase of lager brewing yeast (non-ScTPS2) (SEQ ID NO: 1) was found as a result of a search utilizing the comparison database described in Japanese Patent Application Laid-Open No. 2004-283169. Based on the acquired nucleotide sequence information, primers non-ScTPS2_for (SEQ ID NO: 3) and non-ScTPS2_rv (SEQ ID NO: 4) were designed to amplify the full-length of the gene. PCR was carried out using chromosomal DNA of a genome sequencing strain, Saccharomyces pastorianus Weihenstephan 34/70 (sometimes abbreviated as "W34/70 strain"), as a template to obtain DNA fragments including the full-length gene of non-ScTPS2.

The non-ScTPS2 gene fragments thus obtained were inserted into pCR2.1-TOPO vector (Invitrogen) by TA cloning. The nucleotide sequences of the non-ScTPS2 gene were analyzed by Sanger's method (F. Sanger, Science, 214: 1215, 1981) to confirm the nucleotide sequence.

### Example 2: Analysis of Expression of non-ScTPS2 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

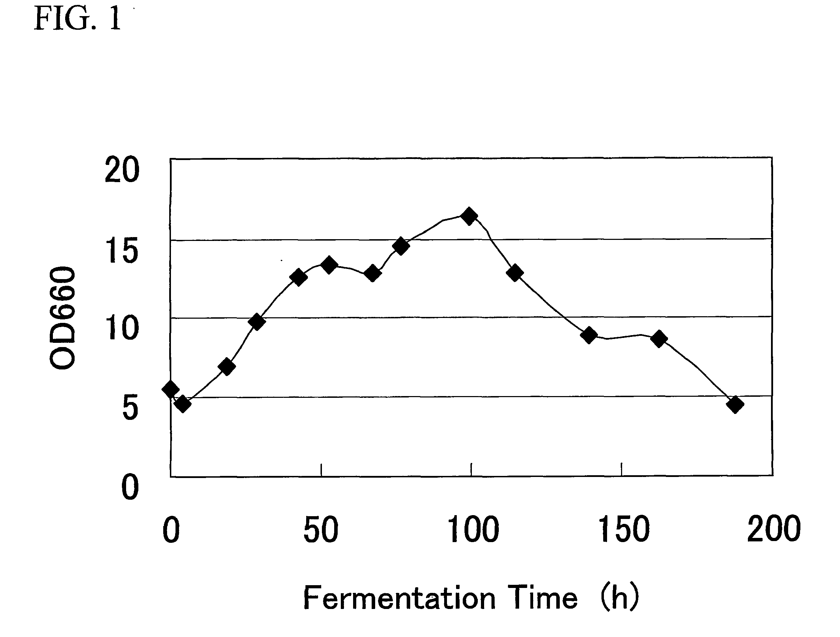

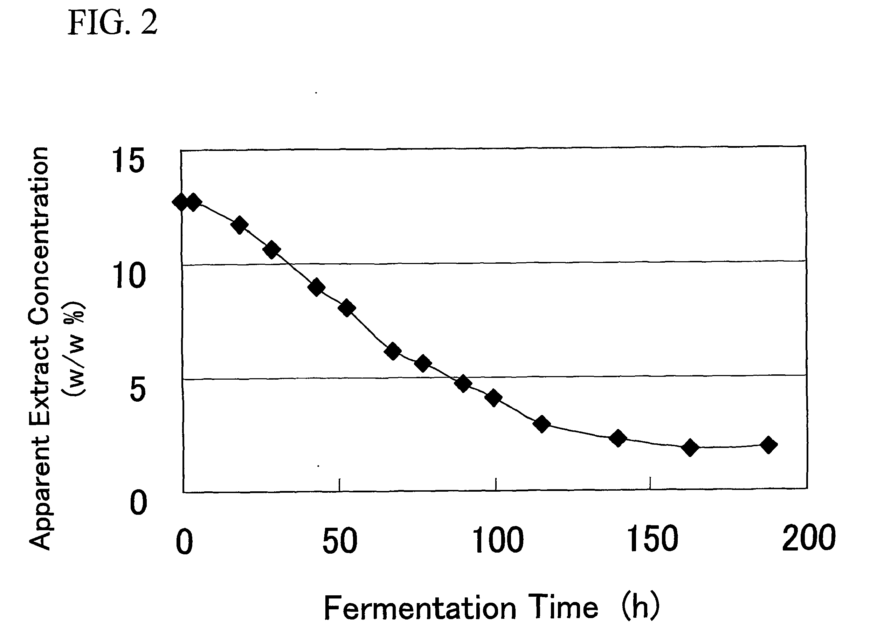

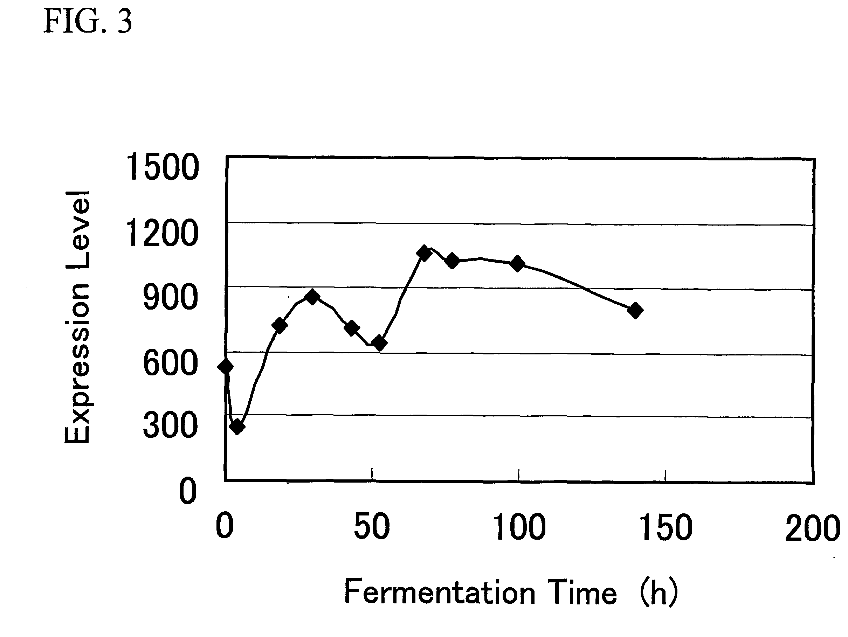

The fermentation liquor was sampled over time, and the time-course changes in amount of yeast cell growth (Fig. 1) and apparent extract concentration (Fig. 2) were observed. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScTPS2 gene is shown in Figure 3. This result confirmed the expression of the non-ScTPS2 gene in the general beer fermentation.

### Example 3: Construction of non-ScTPS2 Highly Expressed Strain

The non-ScTPS2/pCR2.1-TOPO described in Example 1 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScTPS2 high expression vector non-ScTPS2/pYCGPYNot. pYCGPYNot is a YCp-type yeast expression vector. A gene inserted is highly expressed by the pyruvate kinase gene PYK1 promoter. The geneticin-resistant gene G418^{r} is included as the selectable marker in the yeast, and the ampicillin-resistant gene Amp^{r} as the selectable marker in Escherichia coli.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 4: Evaluation of Drying-resistant Property of non-ScTPS2 Highly Expressed Strain

Drying-resistant properties of the parent strain (AJL4004 strain) and the non-ScTPS2 highly expressed strain obtained by the method described in Example 3 were evaluated by a method described below.

One platinum loopful of each yeast was inoculated into 10 mL of wort containing 100 mg/L of geneticin, and stirred at 30°C overnight (precultivation). The precultivation liquid was inoculated into 10 mL wort containing 100 mg/L of geneticin to make its OD660 = 0.5, then main culture was initiated. The culture was continued for 2 days until the growth of the yeast reached stationary phase. Turbidity of the culture was measured at the completion of the culture, then the culture liquid was suspended in sterile water to make its OD = 2. One hundred microliter (100 µL) of the suspension thus obtained was dispensed into a 1.5 mL microtube, then the yeast cells were dried by evaporation for 1 hour using a reduced-pressure concentrator (DNA110 SpeedVac (registered trademark), manufactured by ThermoSavant).

Viable cell ratio was measured by a method described below. The dried yeast cells obtained above were resuspended in 50 µL of sterile water, then 50 µL of 0.02% methylene blue solution (pH 4.5) was added to the suspension. Blue-stained yeast cells which had lost reducing power were considered as dead yeast cells. Then the suspension was observed under a microscope, and viable cell ratio was measured using a Cell Vital Analyzer System (DA cell counter, manufactured by Yamato Scientific Co., Ltd.). The cells were counted until the population reached more than 2000 cells to minimize experimental error.

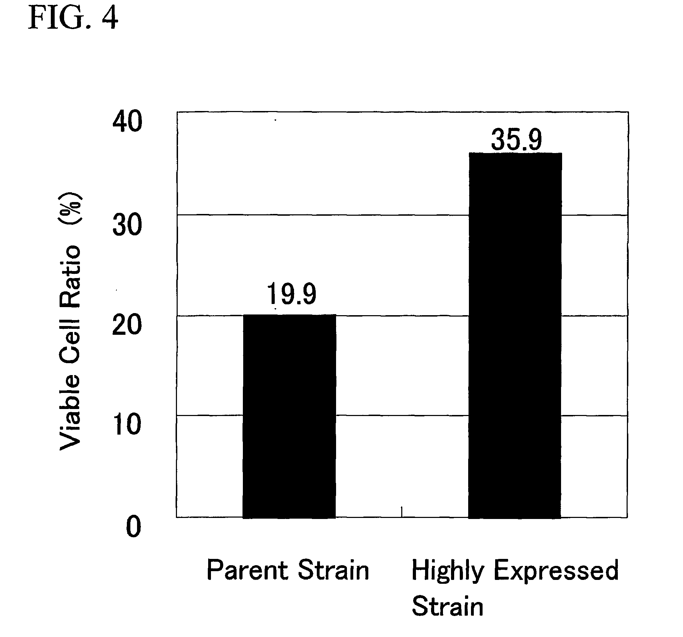

As indicated in Figure 4, viable cell ratio of the highly-expressed strain was 35.9%, though viable cell ratio of the parent strain was 19.9%. It was demonstrated by the results that drying-resistant property of yeast was increased by high expression of non-ScTPS2.

### Example 5: Evaluation of Low-temperature Resistant Property of non-ScTPS2 Highly Expressed Strain

Low-temperature resistant property of the parent strain (AJL4004 strain) and the non-ScTPS2 highly expressed strain obtained by the method described in Example 3 are evaluated by the method described below. Nine hundred microliter (900 µL) of the yeast suspensions cultured by the method described in Example 4 and prepared as OD660=2 are dispensed into two microtubes, respectively. One hundred microliter (100 µL) of sterile water is added to one of the microtubes, on the other hand, 100 µL of 99.5 % ethanol is added to another one (final concentration is 10%). The suspensions are stored at 5°C for 4 weeks, then viable cell ratios are measured by the same method as Example 4.

### INDUSTRIAL APPLICABILITY

According to the present invention, yeast can be stored stably for extended period of time, because drying-resistant property can be enhanced by the present invention. Accordingly, efficiency of brewing alcoholic beverages (such as beer), production of bread, or manufacturing useful materials such as industrial alcohol production or production of useful proteins, etc., can be improved by the present invention.

## Claims

1. A polynucleotide selected from the group consisting of:
(a) a polynucleotide comprising a polynucleotide consisting of the nucleotide sequence of SEQ ID NO: 1;
(b) a polynucleotide comprising a polynucleotide encoding a protein consisting of the amino acid sequence of SEQ ID NO: 2;
(c) a polynucleotide comprising a polynucleotide encoding a protein consisting of the amino acid sequence of SEQ ID NO: 2 in which 1 to 26 amino acids thereof are deleted, substituted, inserted and/or added, and having a trehalose-6-phosphate dephosphorylation activity; and
(d) a polynucleotide comprising a polynucleotide encoding a protein having an amino acid sequence having 97% or higher identity with the amino acid sequence of SEQ ID NO: 2, and said protein having a trehalose-6-phosphate dephosphorylation activity.

2. The polynucleotide according to Claim 1 selected from the group consisting of:
(g) a polynucleotide comprising a polynucleotide encoding a protein consisting of the amino acid sequence of SEQ ID NO: 2, or encoding the amino acid sequence of SEQ ID NO: 2 in which 1 to 8 amino acids thereof are deleted, substituted, inserted, and/or added, and wherein said protein has a trehalose-6-phosphate dephosphorylation activity; and
(h) a polynucleotide comprising a polynucleotide encoding a protein having 99% or higher identity with the amino acid sequence of SEQ ID NO: 2, and having a trehalose-6-phosphate dephosphorylation activity.

3. The polynucleotide according to Claim 1 comprising a polynucleotide consisting of the nucleotide sequence of SEQ ID NO: 1.

4. The polynucleotide according to Claim 1 comprising a polynucleotide encoding a protein consisting of the amino acid sequence of SEQ ID NO: 2.

5. The polynucleotide according to any one of Claims 1 to 4, wherein the polynucleotide is DNA.

6. A protein encoded by the polynucleotide according to any one of Claims 1 to 5.

7. A vector containing the polynucleotide according to any one of Claims 1 to 5.

8. A yeast into which the vector according to Claim 7 has been introduced.

9. The yeast according to Claim 8, wherein drying-resistant property is increased.

10. A method for producing an alcoholic beverage by using the yeast according to Claim 8 or 9.

11. A method for assessing a test yeast for its drying-resistant property, comprising using a primer or probe designed based on the nucleotide sequence of a gene having the nucleotide sequence of SEQ ID NO: 1 and encoding a trehalose-6-phosphate phosphatase.

12. A method for assessing a test yeast for its drying-resistant property, comprising: culturing the test yeast; and measuring the expression level of the gene having the nucleotide sequence of SEQ ID NO: 1 and encoding a trehalose-6-phosphate phosphatase.

13. A method for selecting a yeast, comprising: culturing test yeasts; quantifying the protein of Claim 6 or measuring the expression level of the gene having the nucleotide sequence of SEQ ID NO: 1 and encoding a trehalose-6-phosphate phosphatase; and selecting a test yeast having an amount of the protein or the gene expression level according to favorable drying-resistant property.

14. A method for producing an alcoholic beverage comprising: conducting fermentation using the yeast according to Claim 8 or 9 or a yeast selected by the methods according to Claim 13.

## Patentansprüche

1. Polynucleotid ausgewählt aus der Gruppe bestehend aus:
(a) einem Polynucleotid umfassend ein Polynucleotid bestehend aus der Nucleotidsequenz SEQ ID NO:1;
(b) einem Polynucleotid umfassend ein Polynucleotid, das ein Protein codiert, das aus der Aminosäuresequenz SEQ ID NO:2 besteht;
(c) einem Polynucleotid umfassend ein Polynucleotid, das ein Protein codiert, das aus der Aminosäuresequenz SEQ ID NO:2 besteht, in der 1 bis 26 Aminosäuren davon entfernt, substituiert, eingefügt und/oder hinzugefügt sind, und wobei das Protein eine Trehalose-6-phosphat-Dephosphorylierungsaktivität hat; und
(d) einem Polynucleotid umfassend ein Polynucleotid, das ein Protein codiert, das eine Aminosäuresequenz hat, die eine 97%ige oder höhere Identität mit der Aminosäuresequenz SEQ ID NO:2 hat, und wobei das Protein eine Trehalose-6-phosphat-Dephosphorylierungsaktivität hat.

2. Polynucleotid gemäß Anspruch 1, ausgewählt aus der Gruppe bestehend aus:
(g) einem Polynucleotid umfassend ein Polynucleotid, das ein Protein codiert, das aus der Aminosäuresequenz SEQ ID NO:2 besteht, oder die Aminosäuresequenz SEQ ID NO:2 codiert, in der 1 bis 8 Aminosäuren davon entfernt, substituiert, eingefügt und/oder hinzugefügt sind, und wobei das Protein eine Trehalose-6-phosphat-Dephosphorylierungsaktivität hat; und
(h) einem Polynucleotid umfassend ein Polynucleotid, das ein Protein codiert, das eine 99%ige oder höhere Identität mit der Aminosäuresequenz SEQ ID NO:2 hat, und wobei das Protein eine Trehalose-6-phosphat-Dephosphorylierungsaktivität hat.

3. Polynucleotid gemäß Anspruch 1 umfassend ein Polynucleotid, das aus der Nucleotidsequenz SEQ ID NO:1 besteht.

4. Polynucleotid gemäß Anspruch 1 umfassend ein Polynucleotid, das ein Protein codiert, das aus der Aminosäuresequenz SEQ ID NO:2 besteht.

5. Polynucleotid gemäß einem der Ansprüche 1 bis 4, wobei das Polynucleotid DNA ist.

6. Protein codiert durch das Polynucleotid gemäß einem der Ansprüche 1 bis 5.

7. Vektor, der das Polynucleotid gemäß einem der Ansprüche 1 bis 5 enthält.

8. Hefe, in die der Vektor gemäß Anspruch 7 eingeführt wurde.

9. Hefe gemäß Anspruch 8, wobei das Merkmal Trockenheitsresistenz gesteigert ist.

10. Verfahren zur Herstellung eines alkoholischen Getränks durch die Verwendung der Hefe gemäß Anspruch 8 oder 9.

11. Verfahren zur Bewertung des Merkmals Trockenheitsresistenz einer Testhefe, umfassend die Verwendung eines Primers oder einer Sonde, die basierend auf der Nucleotidsequenz eines Gens entworfen wurden, das die Nucleotidsequenz SEQ ID NO:1 hat und eine Trehalose-6-phosphat-Phosphatase codiert.

12. Verfahren zur Bewertung des Merkmals Trockenheitsresistenz einer Testhefe, umfassend: Züchten der Testhefe; und Messen des Expressionsspiegels des Gens, das die Nucleotidsequenz SEQ ID NO:1 hat und eine Trehalose-6-phosphat-Phosphatase codiert.

13. Verfahren zum Auswählen einer Hefe, umfassend: Züchten von Testhefen, Quantifizieren des Proteins gemäß Anspruch 6 oder Messen des Expressionsspiegels des Gens, das die Nucleotidsequenz SEQ ID NO:1 hat und eine Trehalose-6-phosphat-Phosphatase codiert; und Auswählen einer Testhefe, die eine Menge des Proteins oder den Genexpressionsspiegel hat, entsprechend einem vorteilhaften Merkmal Trockenheitsresistenz.

14. Verfahren zur Herstellung eines alkoholischen Getränks, umfassend: Durchführen einer Fermentation unter Verwendung der Hefe gemäß Anspruch 8 oder 9 oder einer Hefe ausgewählt durch die Verfahren gemäß Anspruch 13.

## Revendications

1. Polynucléotide choisi dans le groupe consistant en :
(a) un polynucléotide comprenant un polynucléotide consistant en la séquence de nucléotides de SEQ ID NO : 1 ;
(b) un polynucléotide comprenant un polynucléotide codant pour une protéine consistant en la séquence d'acides aminés de SEQ ID NO :2 ;
(c) un polynucléotide comprenant un polynucléotide codant pour une protéine consistant en la séquence d'acides aminés de SEQ ID NO :2 dans laquelle 1 à 26 acides aminés sont supprimés, substitués, insérés et/ou ajoutés, et ayant une activité de déphosphorylation de tréhalose-6-phosphate ; et
(d) un polynucléotide comprenant un polynucléotide codant pour une protéine ayant une séquence d'acides aminés ayant 97% ou plus d'identité avec la séquence d'acides aminés de SEQ ID NO : 2, et ladite protéine ayant une activité de déphosphorylation de tréhalose-6-phosphate.

2. Polynucléotide selon la revendication 1, choisi dans le groupe consistant en :
(g) un polynucléotide comprenant un polynucléotide codant pour une protéine consistant en la séquence d'acides aminés de SEQ ID NO :2, ou codant pour la séquence d'acides aminés de SEQ ID NO :2 dans laquelle 1 à 8 acides aminés sont supprimés, substitués, insérés, et/ou ajoutés, et dans lequel ladite protéine a une activité de déphosphorylation de tréhalose-6-phosphate ; et
(h) un polynucléotide comprenant un polynucléotide codant pour une protéine ayant 99% ou plus d'identité avec la séquence d'acides aminés de SEQ ID NO :2, et ayant une activité de déphosphorylation de tréhalose-6-phosphate.

3. Polynucléotide selon la revendication 1 comprenant un polynucléotide consistant en la séquence de nucléotides de SEQ ID NO:1.

4. Polynucléotide selon la revendication 1 comprenant un polynucléotide codant pour une protéine consistant en la séquence d'acides aminés de SEQ ID NO:2.

5. Polynucléotide selon l'une quelconque des revendications 1 à 4, dans lequel le polynucléotide est l'ADN.

6. Protéine codée par le polynucléotide selon l'une quelconque des revendications 1 à 5.

7. Vecteur contenant le polynucléotide selon l'une quelconque des revendications 1 à 5.

8. Levure dans laquelle le vecteur selon la revendication 7 a été introduit.

9. Levure selon la revendication 8, dans laquelle la propriété de résistance au séchage est augmentée.

10. Procédé de production d'une boisson alcoolisée en utilisant la levure selon la revendication 8 ou 9.

11. Procédé pour évaluer une levure de test pour sa propriété de résistance au séchage, comprenant l'utilisation d'une amorce ou d'une sonde conçue sur la base de la séquence de nucléotides d'un gène ayant la séquence de nucléotides de SEQ ID NO : :1 et codant pour une phosphatase de tréhalose-6-phosphate.

12. Procédé pour évaluer une levure de test pour sa propriété de résistance au séchage, comprenant : la culture de la levure de test ; et la mesure du niveau d'expression du gène ayant la séquence de nucléotides de SEQ ID NO:1 et codant pour une phosphatase de tréhalose -6-phosphate.

13. Procédé de sélection d'une levure, comprenant : la culture de levures de test ; la quantification de la protéine selon la revendication 6 ou la mesure du niveau d'expression du gène ayant la séquence de nucléotides de SEQ ID NO :1 et codant pour une phosphatase de tréhalose-6-phosphate ; et la sélection d'une levure de test ayant une quantité de protéine ou le niveau d'expression du gène correspondant à une propriété de résistance au séchage favorable.

14. Procédé de production d'une boisson alcoolisée comprenant : la conduite de la fermentation en utilisant la levure selon la revendication 8 ou 9 ou une levure sélectionnée par les procédés selon la revendication 13.


### SEQUENCE LISTING

<110> Suntory Limited
<120> Gene encoding trehalose-6-phosphate phosphatase and use thereof
<130> PCT06-0141
<150> JP2006-54191
   <151> 2006-02-28
<160> 4
<170> PatentIn version 3.3
<210> 1
   <211> 2691
   <212> DNA
   <213> Saccharomyces sp.
<400> 1
   atgactacca ccgcccaaga caattcccct aaaaggaaac agcgtatcat taactgtgtg      60
   acccaactgc cctacaagat ccagcttggc gaaagcaacg atgattggaa aatatcagcc     120
   accacaggca acagcgcgct atactcctct ttggaatacc tgcagtttga ctctaccgag     180
   tacgaacagc atgtcgttgg ttggactggt gaaatcacaa gaactgaacg cagcctgttt     240
   accaaggagg ctaaggagaa gcctcacgac ctggacgatg accccttgta cctgacaaag     300
   gagcagatca atgggctaac caccactctc caggatcata tgaagtccga aaaagaggca     360
   aagaccgaca atactcctac cacctccgct attaacaacg tccatcctgt ttggctgctt     420
   agaaaaaacc aaagcaggtg gagaaactac gccgagaaag tcatctggcc cactttccac     480
   tacatcctga acccctccaa tgaaggggaa caggaaaaaa actggtggta cgattacgtc     540
   aagttcaacg aagcttacgc ccaaaaaatc ggcgaagttt accagaaggg cgacatcatc     600
   tggattcatg actactacct actactactg ccccaactac tgaggatgaa gttcaatgac     660
   gagtccatca tcattggcta tttccatcac gctccatggc ctagtaacga gtacttccgc     720
   tgtctgcccc gtagaaagca aatcttggac ggtcttgtcg gggcaaatag aatttgcttc     780
   caaaacgaat cgttttcccg tcatttcgta tcgagttgta aaagattact agacgccact     840
   gccaagaaat ccaagaactc ctccgataac gaccagtacc aagtctctgt ttacggaggt     900
   gacgtgttgg tagactcttt acccatcggt gtcaacacca ctcaaatcct aaaggatgcc     960
   ttcactaaag atattgactc taaagttctt tccatcaagc aggcctacca gaacaaaaaa    1020
   attatcattg gtagagaccg tttggactcc gttcgtggtg tcgtgcagaa attgagggct    1080
   tttgaaactt tcttggctat gtaccctgaa tggagagacc aagtcgtgct gatccaggtc   1140
   agcagcccca ccgctaccag gacttctcca caaaccatca aactggaaca gcaggtcaac   1200
   gaactggtta actccatcaa ttctgaatac ggtagtctga acttctcccc cgttcaacac   1260
   tattacatga gaatccccaa ggatgtttac ctgtctctac taagagttgc cgatttatgc   1320
   ttaatcacaa gtgttagaga cggtatgaac accactgctt tggagtacgt caccgttaaa   1380
   tcccacatgt ctaatttctt atgctatggt aatcctctaa ttttaagtga attttctggt   1440
   tcaagtaacg tgttgaagga cgcaatcgtg gtcaacccat gggactcagt cgccgtggcc   1500
   aaatccatca acatggcctt caagctagac aaagaggaga aaaccacttt ggaatcaaag   1560
   ttgtggaatg aagtccccac tatccaggac tggaccaata agtttttgac ctcaataaag   1620
   gaattggctt cctctgatga tgatgtcgaa aggaaaatga cacccgctct caatagacct   1680
   gttcttttgg aaaactataa acaatccaaa cgcagattgt tccttttcga ctatgacggt   1740
   acgttaaccc caattgtcaa ggatcccgct gcggccatcc cctcagcaag actttataca   1800
   attttacaaa aattgtctgc tgatcctcat aaccaaatct ggattatttc aggtcgtgac   1860
   caaaaattct taaacaaatg gctaggtggt aaattacctc aattaggttt aagtgccgaa   1920
   catggttgct tcatgaaaga tgtctcttgt gaagactggg tcaatttgac cgaaaaagtc   1980
   gatatgtcct ggcaagtgcg tgtcaatgaa gtgatggaag aattcaccac aagaacccca   2040
   ggttcattca tcgaaagaaa gaaagttgct ttgacttggc attataggcg tacagtccca   2100
   gagttgggtg aattccacgc caaagaattg aaagagaaac tgctaacatt tactgatgat   2160
   ttcgatttgg aagttatgga tggtaaggct aatatcgaag ttcgtccaag gttcgtcaac   2220
   aagggtgaaa tcgtcaagag attggtttgg caccaacatg gccaaccaca agacatgctg   2280
   aaggggatta gtgaaaaatt acctaaagac gaaatgcctg actttgtatt atgtctaggt   2340
   gatgacttta ccgacgaaga tatgttcagg cagttgaata ccatcgaaac ctgttggaaa   2400
   ggaaaatacc cagatgagaa aaatcaatgg ggcaactatg ggttttatcc agtcactgtt   2460
   gggtccgcat ccaagaaaac tgtggctaag gctcatttaa ccgatcctca acaagttttg   2520
   gaaaccctag gtttacttgt cggcgatgtt tcacttttcc aaagcgcagg tactgtcgac   2580
   ttggattcaa gaggtcatgt aaagaacagt gaaagtagtt tgaagtctaa actcgcatcc   2640
   aaagcttacg tcatgaaaag gtctgcttct tacacaggtg cgaaggttta a            2691
<210> 2
   <211> 896
   <212> PRT
   <213> Saccharomyces sp.
<400> 2
   Met Thr Thr Thr Ala Gln Asp Asn Ser Pro Lys Arg Lys Gln Arg Ile
   1               5                   10                  15
   Ile Asn Cys Val Thr Gln Leu Pro Tyr Lys Ile Gln Leu Gly Glu Ser
               20                  25                  30
   Asn Asp Asp Trp Lys Ile Ser Ala Thr Thr Gly Asn Ser Ala Leu Tyr
           35                  40                  45
   Ser Ser Leu Glu Tyr Leu Gln Phe Asp Ser Thr Glu Tyr Glu Gln His
       50                  55                  60
   Val Val Gly Trp Thr Gly Glu Ile Thr Arg Thr Glu Arg Ser Leu Phe
   65                  70                  75                  80
   Thr Lys Glu Ala Lys Glu Lys Pro His Asp Leu Asp Asp Asp Pro Leu
                   85                  90                  95
   Tyr Leu Thr Lys Glu Gln Ile Asn Gly Leu Thr Thr Thr Leu Gln Asp
               100                 105                 110
   His Met Lys Ser Glu Lys Glu Ala Lys Thr Asp Asn Thr Pro Thr Thr
           115                 120                 125
   Ser Ala Ile Asn Asn Val His Pro Val Trp Leu Leu Arg Lys Asn Gln
       130                 135                 140
   Ser Arg Trp Arg Asn Tyr Ala Glu Lys Val Ile Trp Pro Thr Phe His
   145                 150                 155                 160
   Tyr Ile Leu Asn Pro Ser Asn Glu Gly Glu Gln Glu Lys Asn Trp Trp
                   165                 170                 175
   Tyr Asp Tyr Val Lys Phe Asn Glu Ala Tyr Ala Gln Lys Ile Gly Glu
               180                 185                 190
   Val Tyr Gln Lys Gly Asp Ile Ile Trp Ile His Asp Tyr Tyr Leu Leu
           195                 200                 205
   Leu Leu Pro Gln Leu Leu Arg Met Lys Phe Asn Asp Glu Ser Ile Ile
       210                 215                 220
   Ile Gly Tyr Phe His His Ala Pro Trp Pro Ser Asn Glu Tyr Phe Arg
   225                 230                 235                 240
   Cys Leu Pro Arg Arg Lys Gln Ile Leu Asp Gly Leu Val Gly Ala Asn
                   245                 250                 255
   Arg Ile Cys Phe Gln Asn Glu Ser Phe Ser Arg His Phe Val Ser Ser
               260                 265                 270
   Cys Lys Arg Leu Leu Asp Ala Thr Ala Lys Lys Ser Lys Asn Ser Ser
           275                 280                 285
   Asp Asn Asp Gln Tyr Gln Val Ser Val Tyr Gly Gly Asp Val Leu Val
       290                 295                 300
   Asp Ser Leu Pro Ile Gly Val Asn Thr Thr Gln Ile Leu Lys Asp Ala
   305                 310                 315                 320
   Phe Thr Lys Asp Ile Asp Ser Lys Val Leu Ser Ile Lys Gln Ala Tyr
                   325                 330                 335
   Gln Asn Lys Lys Ile Ile Ile Gly Arg Asp Arg Leu Asp Ser Val Arg
               340                 345                 350
   Gly Val Val Gln Lys Leu Arg Ala Phe Glu Thr Phe Leu Ala Met Tyr
   355 360 365
   Pro Glu Trp Arg Asp Gln Val Val Leu Ile Gln Val Ser Ser Pro Thr
   370 375 380
   Ala Thr Arg Thr Ser Pro Gln Thr Ile Lys Leu Glu Gln Gln Val Asn
   385 390 395 400
   Glu Leu Val Asn Ser Ile Asn Ser Glu Tyr Gly Ser Leu Asn Phe Ser
   405 410 415
   Pro Val Gln His Tyr Tyr Met Arg Ile Pro Lys Asp Val Tyr Leu Ser
   420 425 430
   Leu Leu Arg Val Ala Asp Leu Cys Leu Ile Thr Ser Val Arg Asp Gly
   435 440 445
   Met Asn Thr Thr Ala Leu Glu Tyr Val Thr Val Lys Ser His Met Ser
   450 455 460
   Asn Phe Leu Cys Tyr Gly Asn Pro Leu Ile Leu Ser Glu Phe Ser Gly
   465 470 475 480
   Ser Ser Asn Val Leu Lys Asp Ala Ile Val Val Asn Pro Trp Asp Ser
   485 490 495
   Val Ala Val Ala Lys Ser Ile Asn Met Ala Phe Lys Leu Asp Lys Glu
   500 505 510
   Glu Lys Thr Thr Leu Glu Ser Lys Leu Trp Asn Glu Val Pro Thr Ile
   515 520 525
   Gln Asp Trp Thr Asn Lys Phe Leu Thr Ser Ile Lys Glu Leu Ala Ser
   530 535 540
   Ser Asp Asp Asp Val Glu Arg Lys Met Thr Pro Ala Leu Asn Arg Pro
   545 550 555 560
   Val Leu Leu Glu Asn Tyr Lys Gln Ser Lys Arg Arg Leu Phe Leu Phe
   565 570 575
   Asp Tyr Asp Gly Thr Leu Thr Pro Ile Val Lys Asp Pro Ala Ala Ala
   580 585 590
   Ile Pro Ser Ala Arg Leu Tyr Thr Ile Leu Gln Lys Leu Ser Ala Asp
   595 600 605
   Pro His Asn Gln Ile Trp Ile Ile Ser Gly Arg Asp Gln Lys Phe Leu
   610 615 620
   Asn Lys Trp Leu Gly Gly Lys Leu Pro Gln Leu Gly Leu Ser Ala Glu
   625 630 635 640
   His Gly Cys Phe Met Lys Asp Val Ser Cys Glu Asp Trp Val Asn Leu
   645 650 655
   Thr Glu Lys Val Asp Met Ser Trp Gln Val Arg Val Asn Glu Val Met
   660 665 670
   Glu Glu Phe Thr Thr Arg Thr Pro Gly Ser Phe Ile Glu Arg Lys Lys
   675 680 685
   Val Ala Leu Thr Trp His Tyr Arg Arg Thr Val Pro Glu Leu Gly Glu
   690 695 700
   Phe His Ala Lys Glu Leu Lys Glu Lys Leu Leu Thr Phe Thr Asp Asp
   705 710 715 720
   Phe Asp Leu Glu Val Met Asp Gly Lys Ala Asn Ile Glu Val Arg Pro
   725 730 735
   Arg Phe Val Asn Lys Gly Glu Ile Val Lys Arg Leu Val Trp His Gln
   740 745 750
   His Gly Gln Pro Gln Asp Met Leu Lys Gly Ile Ser Glu Lys Leu Pro
   755 760 765
   Lys Asp Glu Met Pro Asp Phe Val Leu Cys Leu Gly Asp Asp Phe Thr
   770 775 780
   Asp Glu Asp Met Phe Arg Gln Leu Asn Thr Ile Glu Thr Cys Trp Lys
   785 790 795 800
   Gly Lys Tyr Pro Asp Glu Lys Asn Gln Trp Gly Asn Tyr Gly Phe Tyr
   805 810 815
   Pro Val Thr Val Gly Ser Ala Ser Lys Lys Thr Val Ala Lys Ala His
   820 825 830
   Leu Thr Asp Pro Gln Gln Val Leu Glu Thr Leu Gly Leu Leu Val Gly
   835 840 845
   Asp Val Ser Leu Phe Gln Ser Ala Gly Thr Val Asp Leu Asp Ser Arg
   850 855 860
   Gly His Val Lys Asn Ser Glu Ser Ser Leu Lys Ser Lys Leu Ala Ser
   865 870 875 880
   Lys Ala Tyr Val Met Lys Arg Ser Ala Ser Tyr Thr Gly Ala Lys Val
   885 890 895
<210> 3
   <211> 40
   <212> DNA
   <213> Artificial
<220>
   <223> Primer
<400> 3
   gagctcatag cggccatgac taccaccgcc caagacaatt 40
<210> 4
   <211> 42
   <212> DNA
   <213> Artificial
<220>
   <223> Primer
<400> 4
   ggatcctatg cggccgccac atcggctgtc caaaaataaa ac 42